# EUROPEAN PATENT APPLICATION

(11) **EP 1 147 786 A2**
(43) Date of publication of application: **24.10.2001**
(21) Application number: 01303416.0
(22) Date of filing: 11.04.2001
(51) Int. Cl.: A61N 5/06, A61B 18/20

(54) **Radiation delivery method and apparatus**

(30) Priority: 20.04.2000 GB 0009901
(71) Applicant: Photo Therapeutics Limited, Altrincham, Cheshire WA14 1EP (GB)
(72) Inventor: Whitehurst, Colin, Photo-Therapeutics Ltd., Altrincham, Cheshire WA14 1EP (GB)
(74) Representative: Cross, James Peter Archibald

(57) **Abstract**

A fibre optic fabric 1 is described comprising optical fibres 2 woven with a fill material 3 where light is periodically emitted from the optical fibres. Such a fabric provides a flexible light source formable into garments for phototherapy or diagnosis.

## Description

The present invention relates to a method and apparatus for delivering electromagnetic radiation to an affected area of a patient, particularly but not exclusively in the treatment of skin conditions, particularly but not exclusively using photosensitising drugs.

In known high-intensity phototherapy techniques, the affected area is treated using a narrow beam so as to concentrate the radiation onto the affected part. Where the affected part extends over a larger area, the treatment becomes very lengthy. In addition, the geometry of the treated areas can create serious problems in achieving uniform illumination.

Alternative devices have been proposed by which radiation may be applied at multiple points simultaneously. For example, US 5,000,752 (Hoskin) discloses apparatus for treating port wine stains using a pad supporting a plurality of diamond-tipped needles, to which laser radiation is delivered through optical fibres. However, such apparatus is difficult to position correctly, requires the patient to be anaesthetised and is not suitable for treating large, non-planar areas.

The document US 4,234,907 discloses a light-emitting fabric in which optical fibres are part of the weave. Light is emitted from the optical fibres through small scratches that pierce the outer coating. The fabric is said to be suitable for clothing, for example a suit for tanning in which UV light is emitted towards the user.

The document US 4,907,132 discloses a light-emitting panel including one or more layers of woven fibre optic material having disruptions or bends at discrete locations along the length of the fibres to allow light to be emitted therefrom. The panel can be formed as a pad, belt, collar, blanket, strap or other such shape. The panel is said to be suitable for the treatment of jaundice in newborn babies.

The document US 5,339,223 discloses a servo-control for a fibreoptic phototherapy pad of the type disclosed in US 4,907,132.

One aim of the present invention is to allow phototherapeutic treatment of large, non-planar areas of the skin.

An alternative or additional aim of the present invention is to allow treatment of large areas without causing undue discomfort to the patient.

An alternative or additional aim of the present invention is to provide apparatus suitable for treatment of large areas of the skin with incoherent or non-laser light.

According to one aspect of the present invention, there is provided a garment incorporating optical fibres connectable to a light source, and arranged such that light emitted from the fibres is directed towards a wearer.

According to another aspect of the present invention, there is provided a fibre optic fabric comprising a reflecting portion, a fibre optic portion including optical fibres and a diffusing portion, said fibre optic portion being positioned between said diffusing portion and said reflecting portion.

According to yet another aspect of the present invention, there is provided a fabric comprising a series of woven optical fibres, where the weaving causes any light transmitted through the optical fibres to escape from the fibre.

According to a further aspect of the invention there is provided fabric comprising a series of optical fibres treated so as to cause light transmitted through the optical fibres to substantially continuously escape along the length of the fibre.

According to a further aspect of the invention, there is provided use of a fabric or garment as described above for therapy or cosmetic treatment or photodynamic therapy.

Preferably, the garment or fabric includes a reflective layer positioned outside a layer of optical fibres, so as to reflect escaped light inwards.

Preferably, the garment or fabric includes a diffusing layer positioned inside the layer of optical fibres, so as to diffuse light emitted inwards.

Preferably, the garment or fabric includes means for coupling incoherent light from a light source into the optical fibres.

The garment or fabric may have a plurality of layers incorporating optical fibres.

The invention will now be described with reference to the following drawings in which:
Figure 1 is a cross section through one form of fibre optic layer in accordance with the invention, showing the path of the light transmitted through an individual optical fibre;
Figure 2 is an exploded perspective view of one form of material in accordance with the invention, incorporating a layer as shown in Figure 1, showing connection of the optical fibres to a suitable light source;
Figure 3 is an exploded perspective view of a second embodiment of the invention, showing a further form of material including layers comprising side emitting optical fibres; and
Figure 4 is a schematic perspective view of a further embodiment of the invention, showing a plurality of fibre optic layers, as shown in Figure 1, attached together to form a larger blanket.

Light can be transmitted through optical fibres by total internal reflection; that is, any light rays travelling through the fibre and incident on the internal walls of the fibre at angles greater than a critical angle to the perpendicular (where the critical angle = sin⁻¹.(1/n) where n is the refractive index of the fibre medium) are reflected back into the optical fibre.

Weaving such optical fibres together with suitable fill material forms a flexible fibre optic fabric layer 1 having advantageous properties in the field of PDT.

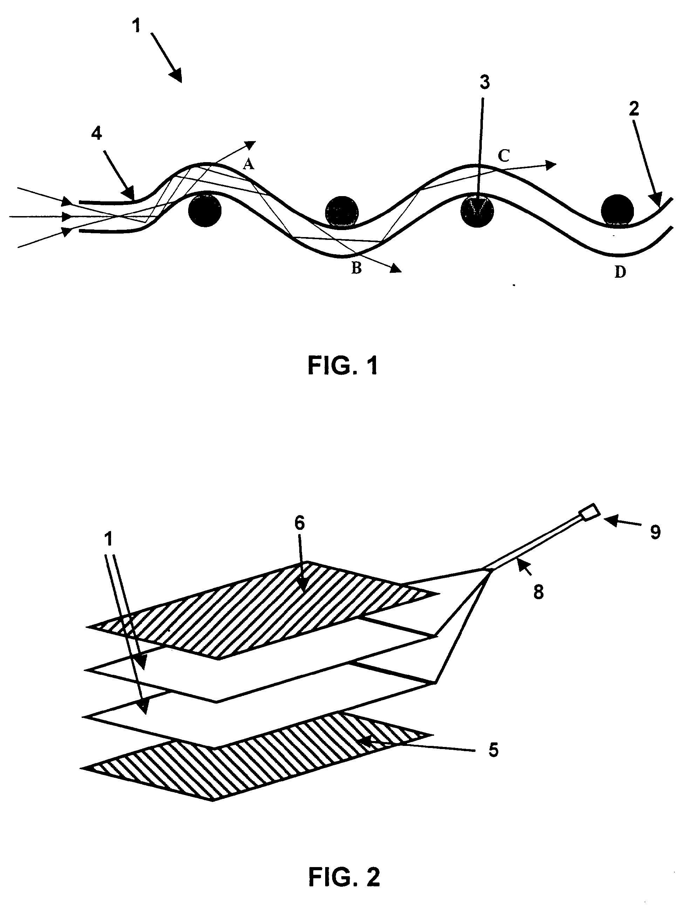

Figure 1 illustrates a cross-section through such a flexible fabric layer 1 showing a section through one optical fibre 2 interwoven with several fill fibres 3.

As, in this case, the optical fibre 2 is bent around fill fibres 3, certain of the light rays transmitted through the optical fibre 2 will not be incident on the internal walls of the optical fibre 2 at angles greater than the critical angle, and as such will be refracted out of the optical fibre 2 and hence out of the layer of fabric 1.

As can be seen in Figure 1, at points A, B, C and D, the light travelling through the optical fibre 2 is emitted from the side of the fibre 2 through cladding 4 on the optical fibre 2. It will be appreciated that when a large number of optical fibres 2 are woven in this way, the optical fibre fabric layer 1 formed will emit light in a generally uniform distribution across the fabric layer 1.

Referring to Figure 2, two fibre optic fabric layers 1, as described above and shown in Figure 1, are positioned between a diffusing layer 5 and a reflecting layer 6 to form a flexible multi-layered material suitable for use in PDT applications.

The diffusing layer 5 may be formed from a thin sheet of translucent plastic material that further diffuses the light emitted from the optical fibres 2. However, any suitable form of diffusing material may be used. The diffusing layer 5 will also act to protect the fibre optic fabric layer.

As the light emitted from the optical fibre fabric layers 1 is generally of a uniform distribution when the diffusing layer 5 is not present, it is not essential that a diffusing layer 5 be included.

A layer of transparent plastic material may be provided on the side of the optical fibre fabric layer 1 in place of the diffusing layer 5 to prevent damage on handling. The transparent plastic layer on the innermost surface of the optical fibre fabric layer 1 protects the optical fibres 2 in the fibre optical layer 1 when it is placed in contact with a patient undergoing PDT.

The reflecting layer 6 is a diffusely or specularly reflective layer provided on the surface of the optical fibre fabric layer 1 opposing the surface carrying the diffusing or protecting layer 5. The reflecting layer 6 acts to reflect light emitted towards the reflecting layer 6, from the fibre optic fabric layers 1, back towards the fibre optic fabric layers 1 and the diffusing layer 5.

The reflecting layer 6 may be formed from a Mylar (TM) diffuse reflecting sheet, a self-adhesive metallic vinyl sheet, such as DMX300 supplied by X-Film UK, Luton, Bedfordshire, or a thin aluminised or metallised plastic sheet.

Although the fibre optic fabric material shown in Figure 2 includes two fibre optic fabric layers 1, it will be appreciated that any number of fibre optic fabric layers 1 may be used. Indeed, a single fibre optic fabric layer 1 may suffice.

The ends of the optical fibres 2 of the fibre optic layers 1 are brought together and formed into a fibre optic bundle 8. The ends are then terminated, polished and fixed, for example by adhesive, into a connector 9 for connection to a suitable PDT light source (not shown) emitting, in this case, non-laser or incoherent light. One example of a suitable light source is disclosed in UK Patent No 2272278 and is hereby incorporated in its entirety. The light source may have had the ultra-violet, infrared and non-active visible light removed, however, any suitable light source may be used. Depending on the number of optical fibres 2 involved, the diameter of the face of the bundle 8 may be in the region of 5 to 20 mm. This makes the material I particularly suited to connection to a non-laser light source, as these sources have an inherently large focused beam diameter of at least 5 mm.

Should a suitably small fibre optic bundle 8 be formed, it will be appreciated that the fibre optic fabric layers 1 described here may also be connected to a laser light source.

The optical fibres 2 are preferably formed from polymethyl methacrylate (PMMA) which has the appropriate transmission properties. However, it will be appreciated that any suitable glass material, such as E-glass, or plastic material having the appropriate light transmission properties may be used. The optical fibres are preferably clad but it will be appreciated that cladding is not essential.

Preferably, the diffusing layer 5 (if in use), the fibre optic layers 1 and the reflecting layer 6 are connected together at the edges by adhesive. However, it is not always necessary to attach the layers together at all. Furthermore, other suitable methods of attachment may be used such as stitching. Additionally, the layers need no be attached together at their edges but may be attached at any other suitable location.

In use, the material 1 is placed on a patient with the diffusing layer 5 closest to the patient and the light is transmitted from the PDT light source into the optical fibres 2 of the fibre optic fabric layers 1. The light is transmitted along the axis of the optical fibres 2 and periodically escapes from the top and bottom surface of the fibre optic fabric layer 1.

As shown in Figure 1, the light escaping from the bottom surface, at points B and D, is transmitted toward the diffusing layer 5 and hence towards the patient. The light escaping from the top surface, at points A and C, is transmitted toward the reflecting layer 6 where it is reflected back toward the fibre optic layer 1, the diffusing layer 5 and hence the patient.

It will be appreciated that the fabric as shown in Figure 2 is drawn in an exploded manner and that the diffusing layer 5, the optical fibre layers 1 and the reflecting layer 6 when in use appear as a single multi-layer flexible material. However, further layers of material may be positioned between the reflecting layer 6, the fibre optic layers 1 and the diffusing layer 5.

As the flexible material 1 is woven from elastically deformable fibres 2, a flexible light source is provided that could be manufactured to fit a wearer. For example, the fabric could be formed into the shape of a glove, a sleeve, a collar, or jacket or a large blanket for uniformly illuminating large non-planar areas of a wearer whilst at the same time maintaining wearer mobility and comfort.

Such a fabric or garment would provide a flexible PDT source for treatment of skin conditions or for use with photosensitising drugs easily and comfortably wearable by any person. Fabrics or garments manufactured for such use would require the material to be arranged such that the diffusing layer 5 is positioned closest to the wearer, the fibre optic layer or layers 1 are positioned outwardly of the diffusing layer 5 relative to the wearer, and the reflecting layer 6 is positioned outwardly of the fibre optic layer or layers 1 relative to the wearer.

It will be appreciated that parameters such as flexibility, number of fibre optic layers and size of illuminating area need to be carefully chosen when the fabric is used for PDT. Different forms of treatment for different areas of the body will require different forms of fibre optic material 1. The flexibility of the material 1 depends on the diameter of the individual optical fibres, the material used for the diffusing layer, the thickness and rigidity of the diffusing layer and the ply or number of woven fibre optic fabric layers 1 employed

For example, if a highly irregular or tightly curved area of the body is to be treated, a high degree of flexibility in the fibre optic material 1 is required. For these curved areas, the material 1 is formed using optical fibres 2 having a diameter of between 200 and 250 µm and between one and three fibre optic fabric layers 1. No diffusing layer 5 is included. Such a material 1 is highly flexible. For more planar areas of the body, where the material 1 does not have to be as flexible, more fibre optic fabric layers may be used, for example between three and six layers 1. Alternatively or additionally, optical fibres 2 having a larger diameter of around 300 µm may be used. A diffusing layer 5 may be employed.

The output intensity of the material 1 can be increased by increasing the optical power launched into the fibre optic bundle 8, by increasing the number of fibre optic fabric layers 1 or by a combination of increased power and increased number of layers 1.

The use of plastic optical fibres 2 can limit the optical power in the material 1. Increasing the optical power launched into the fibre optic bundle 8 by too large an amount may cause damage to the optical fibres 2 and may cause the connection between the fibre optic bundle 8 and the light source 10 to be damaged. For example melting of the fibres 2 may occur. The maximum power that can be used will depend on the ambient temperature, the degree of infrared filtration and the wavelength of the light used. In any case, the temperature of the fibre optic bundle 8 should not exceed 85° C.

Preferably, the light launched into the optical fibre has the ultra-violet and infrared portions filtered out. The light may also have any other non-active light removed. In this way, wastage of light and glare are reduced. This also prevents the fibres, and hence the material and the patient, from heating up substantially.

Furthermore, as the light is transmitted from the light source via light guides, the light source can be positioned remote from the patient and there is little electromagnetic (EM) radiation emitted transmitting the light via the fibre optic bundle 8 to the fibre optic fabric material in the vicinity of the patient. EM radiation can adversely affect heart pacemakers and other electronic equipment in use in hospitals. The fibre optic fabric material provides a flexible long-life PDT source that can be moulded to uneven contours, like an item of clothing.

In general, therefore, to produce a uniform output, between two and four fibre optic fabric layers 1 should be used. Loss of flexibility and self-absorption of emitted light yields an upper limit of about six fibre optic fabric layers 1.

The size of the fabric panel to be used will also depend on the treatment being carried out. For example, a panel for wrapping around and treating the whole or partial circumference of a leg would require a surface approximately 15 cm by 60 cm. However, the dimensions of the active, light emitting area of the material 1 will vary depending on the size and number of lesions to be treated and the size of the patient's leg. A Velcro (TM) attachment would be provided for securing the material to the patient's body. Similarly, for treatment of an arm, a scaled down version of the above would be required.

For treatment of a torso, a scaled up version would be required. However, the material 1 could be formed into a garment such as a waistcoat for treatment of the front and back of the torso. This would advantageously secure the material 1 to the patient during treatment. Furthermore, it will be appreciated that other garments such as gloves, jackets or collars could be formed from the material 1 for treatment of appropriate parts of the body.

In a second embodiment of the invention, the fibre optic fabric layers 12 are formed from optical fibres 14 that are placed adjacent and in parallel to one another. These optical fibres 14 have surfaces that have been roughened, for example by chemical alteration such as etching or by rubbing their surfaces with glass paper. This changes the transmission properties of the optical fibres 14 and causes them to emit light from their surfaces along their length within the fibre optic fabric layer 1 in a manner similar to that described above with reference to the first embodiment of the invention. Fibres suitable for use in this way are supplied by Intelite, Inc., CA, USA. However, it will be appreciated that any suitable optical fibres may be used.

The optical fibres 14 are again brought together into one bundle 8 and connected to a suitable PDT light source for use in PDT as described above. Furthermore, the diffusing layer 5 and reflecting layer 6 may be included as described above.

In this way, a fibre optic layer for inclusion in a fibre optic fabric suitable for PDT may be formed without weaving optical fibres together with fill fibres.

The fabric formed using the fibre optic layers described in relation to the second embodiment of the invention, is used in the same manner described above in relation to the first embodiment of the invention.

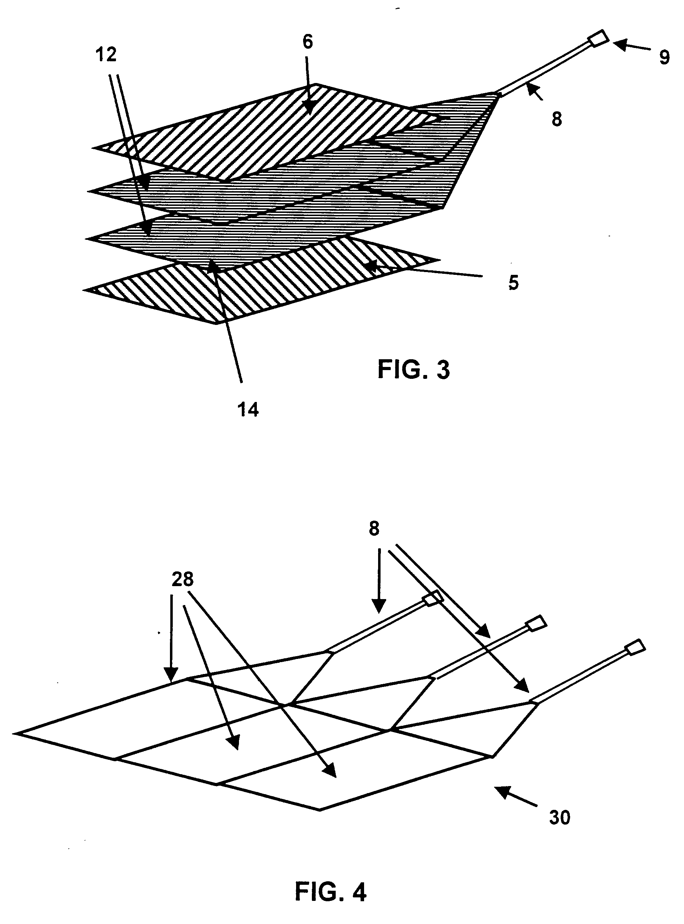

In a further embodiment of the invention, as shown in Figure 4, panels 28 including fibre optic layers 1, previously described above, are attached together to form a large blanket 30. Preferably, the layer or layers 1 of each panel 28 have their own fibre optic bundle 8 connected to a suitable PDT light source. In use, therefore, it is possible to activate only a portion of the whole blanket and treatment can be aimed only where required.

Similarly, fibre optic layers 1 or panel 28 could be incorporated into garments having non-active portions: for example, a waistcoat or jacket where the torso portion is formed from panels 28 or fibre optic layers 1 but the shoulder and/or sleeve portions are formed from non-fibre optic material. In this manner, the patient can easily wear the garment, whilst the panels 28 or fibre optic layers 1 are positioned correctly for treatment purposes.

A method of treatment for superficial cases of actinic/solar keratoses, Bowen's disease, superficial basal cell carcinoma, squamous cell carcinoma, intraepithelial carcinoma, mycosis fungoides, T-cell lymphoma, acne and seborrhoea, psoriasis, eczema, nevus sebaceous, viral infections such as herpes simplex, molluscum contagiosum, and warts (recalcitrant, verruca vulgaris or verruca plantaris), alopecia areata or hirsutism, using a fibre optic fabric material as detailed above, will now be described.

A cream or solution containing a photosensitising drug such as 5-ALA is applied topically under medical supervision to the affected area of the skin of the patient, or administered intravenously or orally. For large areas, the patient may be immersed in a bath of solution. The affected area may then be covered for a period of 3 to 6 hours, or up to 24 hours if the treatment is to be continued the next day, to prevent removal of the drug and carrier, or activation by sunlight. The area is then uncovered and exposed to light from the light source, via the fibre optic fabric material, as described in any of the embodiments for a period of 15 to 30 minutes. The treatment may then be repeated as necessary, for a total of 1 to 3 treatments or more, depending on the severity and type of the condition. This method is particularly suitable for the treatment of patients with very large lesions or multiple lesions extending over a large area.

The light source may also be used for fluorescence detection (photodiagnosis).

The light source may also be used for cosmetic or partially cosmetic treatment with a photosensitizing drug for portwine stain removal and hair restoration/removal, and without a photosensitizing drug for skin rejuvenation, wrinkle removal or biostimulation (including wound healing).

The above embodiments are described purely by way of example and variants or alternatives may be envisaged which nevertheless fall within the spirit or scope of the present invention.

## Claims

1. A wearable garment of woven material incorporating optical fibres connectable to a light source and woven such that light is emitted from the optical fibres towards a wearer.

2. A garment according to claim 1 in which said optical fibres form at least one layer of the garment.

3. A garment according to claim 2, further comprising a reflecting layer positioned outwardly of the fibre optic layer or layers relative to the wearer so as to reflect any outwardly emitted light back toward said wearer.

4. A garment according to any preceding claim, wherein the diameter of the optical fibres is between 200 and 250 µm.

5. A garment according to claim 2 or 3, further comprising a diffusing layer positioned inwardly of the fibre optic layer or layers relative to the wearer so as to diffuse said light directed toward the wearer.

6. A fibre optic fabric comprising a reflecting layer, one or more fibre optic layers each including optical fibres, and a diffusing layer, said fibre optic layer being positioned between said diffusing layer and said reflecting layer.

7. A fibre optic fabric according to claim 6, in which said fabric is formed in the shape of a wearable garment.

8. A fibre optic fabric according to claim 7, in which said diffusing layer is positioned inwardly of said fibre optic layer relative to a wearer of the garment.

9. A fibre optic fabric according to claim 7 or 8, in which said reflecting layer is positioned outwardly of said fibre optic portion relative to the wearer of the garment.

10. A fibre optic fabric according to any one of claims 7 to 9, in which said reflecting portion acts so as to reflect any outwardly transmitted light inwardly toward said wearer.

11. A fibre optic fabric according to any one of claims 7 to 10, in which said diffusing portion acts so as to diffuse any inwardly transmitted light directed toward said wearer.

12. A fabric or garment according to any preceding claim, in which the optical fibres are connected to a light source.

13. A fabric or garment according to claim 12, in which said light source comprises an incoherent or non-laser light source.

14. A fabric or garment according to any preceding claim in which said optical fibres are formed from PMMA.

15. A fabric or garment according to any preceding claim in which said optical fibres are interwoven with fill fibres.

16. A fabric or garment according to any of claims 3 to 13 and 14 and 15 when dependent thereon, in which said reflecting layer is formed from Mylar (TM).

17. A fabric including optical fibres treated so as to cause light transmitted through the optical fibres to escape substantially continuously along the length of the fibres.

18. A fabric according to claim 17, in which the treatment comprises chemical treatment of the surface of the fibres.

19. A fabric according to claim 17, in which the treatment comprises abrasion of the surface of the fibres.

20. Use of a fabric or garment according to any preceding claim, for therapy of a patient.

21. Use of a fabric or garment according to any one of claims 1 to 18 for cosmetic treatment of a patient.

22. Use of a fabric or garment as claimed in claim 20 or 21, for photodynamic therapy of said patient.

23. Use as claimed in any one of claims 20 to 22 in the treatment of one or more of: actinic/solar keratoses, Bowen's disease, superficial basal cell carcinoma, squamous cell carcinoma, intraepithelial carcinoma, mycosis fungoides, T-cell lymphoma, acne and seborrhoea, psoriasis, eczema, nevus sebaceous, viral infections such as herpes simplex, molluscum contagiosum, and warts (recalcitrant, verruca vulgaris or verruca plantaris), alopecia areata or hirsutism.

24. Use according to claim 23, wherein the condition is superficial.

25. Use according to any one of claims 20 to 24, wherein the affected area of the patient is treated with PpIX.

26. A method of cosmetic treatment of a human or animal body, comprising:
applying a photosensitizer to the area to be treated, and
illuminating the area with light from a garment or fabric according to any one of claims 1 to 19.

27. A method of medical treatment of a human or animal body, comprising:
applying a photosensitizer to the area to be treated, and
illuminating the area with light from a garment or fabric according to any one of claims 1 to 19.
